# EUROPEAN PATENT APPLICATION

(11) **EP 3 312 651 A1**
(43) Date of publication of application: **25.04.2018**
(21) Application number: 16811718.2
(22) Date of filing: 16.06.2016
(51) Int. Cl.: G02B 7/02, A61B 1/00, A61B 1/04, G02B 23/26

(54) **LENS UNIT AND ENDOSCOPE**

(30) Priority: 16.06.2015 JP 2015121159
(71) Applicant: Olympus Corporation, Hachioji-shi Tokyo 192-8507 (JP)
(72) Inventor: OGAWA, Tomoaki, Hachioji-shi, Tokyo 192-8507 (JP)
(74) Representative: Schicker, Silvia
(86) International application number: PCT/JP2016/067976
(87) International publication number: WO 2016/204244

(57) **Abstract**

A lens unit (5) according to the present invention includes a plurality of lenses, a lens holding frame (50) having a tubular shape and configured to hold the plurality of lenses inside thereof, a step portion provided in any of the plurality of lenses and having a stepped shape along a direction of an optical axis of the lens, and a convex portion protruding from an inner circumferential surface of the lens holding frame (50) into a space formed by the inner circumferential surface of the lens holding frame (50) and the step portion, in accordance with a position of the lens in which the step portion is formed.

## Description

### Field

The present invention relates to a lens unit and an endoscope.

### Background

Conventionally, in the medical field, an endoscopic system is used to observe the inside of a subject, such as a patient. The endoscopic system includes, for example, an endoscope having an insertion portion on which an image sensor is provided and which is inserted into the subject, and an image processing apparatus connected to the endoscope via a cable and configured to perform image processing on an in-vivo image corresponding to an imaging signal generated by the image sensor and to display the in-vivo image on a display unit or the like.

The endoscope includes an imaging system formed by a combination of an image sensor, such as a charge coupled device (CCD), and a lens unit including a plurality of lenses to generate an endoscopic image (for example, see Patent Literature 1). The lens unit is constituted by a single lens, a doublet lens in which a plurality of lenses are bonded together, a spacer member (spacer ring), a lens holding frame that holds the lens, the doublet lens, and the spacer ring, and the like in such a manner that the lenses are dropped into the lens holding frame and fixed by bonding in a state where an interval between the lenses is maintained with the spacer ring interposed between the lenses.

### Citation List

### Patent Literature

Patent Literature 1: JP 2002-207179 A

### Summary

### Technical Problem

However, the lens holding frame is generally made by using metal, and it is difficult to check positions and orientations of the lenses and the spacer ring after the plurality of lenses and the spacer ring are dropped into the lens holding frame. For example, when a lens in which one end face (front) and the other end face (back) have substantially symmetric shapes in an optical axis direction is to be dropped, it is difficult to check the orientation of the lens from outside, and therefore, there may be a case where the lens is dropped with the front and back sides upside down by mistake and a lens unit is erroneously assembled. In this case, if the erroneously assembled lens unit is assembled in the endoscope, an assembly error of the lens unit is not found until a defect is detected in images checked after completion of the endoscope. In addition, if the assembly error is found after the completion of the endoscope, it is necessary to decompose the endoscope and reassemble the lens unit.

The present invention has been made in view of the foregoing, and an object of the invention is to provide a lens unit and an endoscope capable of easily determining an assembly error of the lens during assembly.

### Solution to Problem

To solve the above-described problem and achieve the object, a lens unit according to the present invention includes: a plurality of lenses; a lens holding frame having a tubular shape and configured to hold the plurality of lenses inside thereof; a step portion provided in any of the plurality of lenses and having a stepped shape along a direction of an optical axis of the lens; and a convex portion protruding from an inner circumferential surface of the lens holding frame into a space formed by the inner circumferential surface of the lens holding frame and the step portion, in accordance with a position of the lens in which the step portion is formed.

In the above-described lens unit according to the present invention, a length of the convex portion corresponding to the direction of the optical axis is shorter than a length of a step of the step portion in the direction of the optical axis.

In the above-described lens unit according to the present invention, a protruding length of the convex portion from the inner circumferential surface of the lens holding frame is equal to or shorter than a height of a step of the step portion in a direction perpendicular to the optical axis.

In the above-described lens unit according to the present invention, the convex portion is provided so as to be freely inserted in and removed from an opening portion formed on the lens holding frame.

In the above-described lens unit according to the present invention, the lens includes a plurality of the step portions.

In the above-described lens unit according to the present invention, the convex portion is formed in a certain position in accordance with a lens that is arranged on an end portion at a side different from an opening end side of the lens holding frame in a lens group formed of the plurality of lenses and spacer rings provided between adjacent ones of the lenses.

An endoscope according to the present invention is an endoscope having an insertion portion inserted into a subject, the endoscope including: the lens unit according to the present invention; and an image sensor configured to receive light that has passed through the lens unit and convert the light into an electrical signal.

### Advantageous Effects of Invention

According to the present invention, it is possible to easily determine an assembly error of a lens during assembly.

### Brief Description of Drawings

FIG. 1 is a diagram schematically illustrating an endoscopic system according to an embodiment of the present invention.
FIG. 2 is a perspective view schematically illustrating a configuration of a lens unit of the endoscopic system according to the embodiment of the present invention.
FIG. 3 is a partial cross sectional view schematically illustrating the configuration of the lens unit of the endoscopic system according to the embodiment of the present invention.
FIG. 4 is a perspective view schematically illustrating a configuration of a main part of the lens unit of the endoscopic system according to the embodiment of the present invention.
FIG. 5 is a diagram viewed from a direction of arrow A illustrated in FIG. 4.
FIG. 6 is a perspective view schematically illustrating a configuration of a main part of the lens unit of the endoscopic system according to the embodiment of the present invention.
FIG. 7 is a schematic diagram for explaining a configuration of a main part of the lens unit of the endoscopic system according to the embodiment of the present invention.
FIG. 8 is a partial cross sectional view schematically illustrating a configuration of the lens unit of the endoscopic system according to the embodiment of the present invention, and is a diagram illustrating a case of an assembly error.
FIG. 9 is a perspective view schematically illustrating a configuration of a main part of a lens unit of an endoscopic system according to a modification of the embodiment of the present invention.

### Description of Embodiments

Modes for carrying out the present invention (hereinafter, referred to as "embodiments") will be described below with reference to the accompanying drawings.

### (Embodiment)

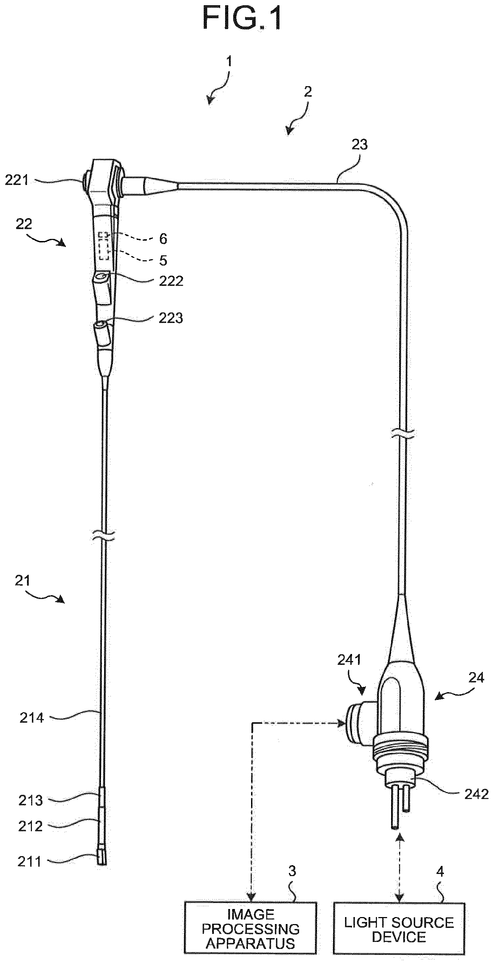

FIG. 1 is a diagram schematically illustrating an endoscopic system 1 according to an embodiment of the present invention. The endoscopic system 1 is a system that applies ultrasound waves to a subject, such as a body wall inside the subject, and generates an image from the reflected ultrasound waves by using the characteristics of the ultrasound waves. As illustrated in FIG. 1, the endoscopic system 1 includes an endoscope 2, an image processing apparatus 3, and a light source device 4. The endoscope 2 is an ultrasound endoscope that has a portion insertable into the subject and a function to transmit ultrasound waves inside the body cavity of the subject, to receive reflected echoes (ultrasound echoes) reflected from the subject, and to output an echo signal. The endoscope 2 is connectable to, via cables, the image processing apparatus 3 that receives the echo signal and generates an ultrasound image corresponding to the echo signal, and the light source device 4 that has a light source for illumination.

The endoscope 2 includes, at a distal end portion thereof, an ultrasound transducer 211 that converts an electrical pulse signal received from the image processing apparatus 3 into an ultrasound pulse (acoustic pulse), applies the ultrasound pulse to the subject, converts an ultrasound echo reflected from the subject into an electrical echo signal represented by a voltage change, and outputs the electrical echo signal. The ultrasound transducer 211 may be any of a convex transducer, a linear transducer, and a radial transducer. The endoscope 2 may be configured to cause the ultrasound transducer 211 to perform mechanical scan, or may include, as the ultrasound transducer 211, a plurality of elements in an array pattern and may cause the ultrasound transducer 211 to perform electrical scan by electrically switching the elements for transmission and reception or by imposing delay on the transmission and reception at each of the elements.

As illustrated in FIG. 1, the endoscope 2 includes an insertion portion 21, an operating unit 22, a universal code 23, and a connector 24. A "distal end side" described herein means a distal end side of the insertion portion 21. In addition, a "proximal end side" described herein means a side separated from the distal end of the insertion portion 21.

The insertion portion 21 is a portion to be inserted into a subject. As illustrated in FIG. 1, the insertion portion 21 includes the ultrasound transducer 211 that is provided at the distal end side, a rigid member 212 that is coupled to a proximal end side of the ultrasound transducer 211, a bending portion 213 that is bendable and coupled to a proximal end side of the rigid member 212, and a flexible tube portion 214 that is flexible and coupled to a proximal end side of the bending portion 213.

While specific illustration is omitted, the insertion portion 21 internally includes a light guide for transmitting illumination light supplied from the light source device 4, a plurality of signal cables arranged for transmitting various signals, and a treatment tool insertion channel for inserting a treatment tool.

The operating unit 22 is a portion that is coupled to the proximal end side of the insertion portion 21 and receives various operations from a doctor or the like. Examples of the operations include an angle operation, an air/water supply operation, and a suction operation. As illustrated in FIG. 1, the operating unit 22 includes a bending knob 221 for performing a bending operation of the bending portion 213. In addition, the operating unit 22 has a treatment tool insertion port 222 that communicates with a treatment tool insertion channel formed inside the insertion portion 21 and that is used for inserting a treatment tool in the treatment tool insertion channel, and an air/water port 223 that communicates with an air/water channel formed inside the insertion portion 21 and that is used for circulating air or liquid in the air/water channel.

The endoscope 2 includes an imaging system including an imaging optical system (a lens unit 5 to be described later) and an image sensor 6, is inserted into a digestive tract (esophagus, stomach, duodenum, or large intestine) or a respiratory organ (trachea or bronchus) of a subject, and is capable of capturing an image of the digestive tract, the respiratory organ, and an organ surrounding the digestive tract or the respiratory organ (pancreas, gallbladder, bile duct, biliary tract, lymph node, mediastinal organ, blood vessel, or the like). The imaging optical system and the image sensor 6 are provided inside the operating unit 22 for example, and receives observation light collected by an objective lens (not illustrated) provided at a distal end of the insertion portion 21 via an optical fiber inserted in the insertion portion 21.

The universal code 23 is a cable which extends from the operating unit 22 and in which a plurality of signal cables for transmitting various signals, optical fibers for transmitting illumination light supplied from the light source device 4, and the like are arranged.

The connector 24 is provided at the distal end of the universal code 23. The connector 24 includes a first connector portion 241, to which a connector portion of a scope cable holding a plurality of signal cables is connected to connect to the image processing apparatus 3 described above, and a second connector portion 242, to which a cable provided with an optical fiber or the like is connected to connect to the light source device 4.

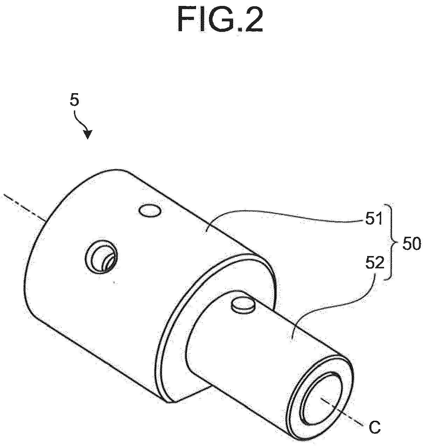

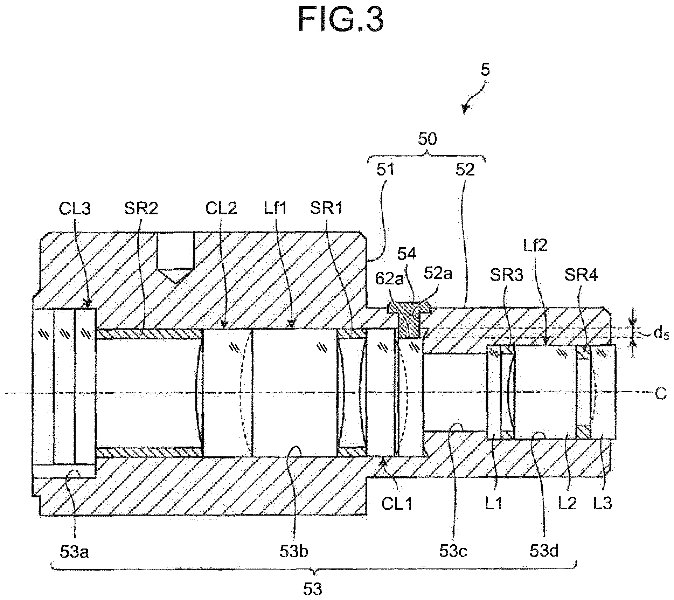

A configuration of the lens unit as the imaging optical system will be described below. FIG. 2 is a perspective view schematically illustrating a configuration of the lens unit 5 of the endoscopic system 1 according to the embodiment of the present invention. FIG. 3 is a partial cross sectional view schematically illustrating the configuration of the lens unit 5 of the endoscopic system 1 according to the embodiment of the present invention, and is a partial cross sectional view of a portion corresponding to a cross section taken along a plane passing through an optical axis C of a lens held by the lens unit.

The lens unit 5 includes a lens holding frame 50, lenses L1 to L3, doublet lenses CL1 to CL3, spacer rings SR1 to SR4, and a pin 54. Each of the spacer rings SR1 to SR4 has a tubular shape and serves as a spacer that maintains an interval between the lenses to be a certain interval for ensuring an optimal optical path and an optimal image magnification. In the embodiment, the lens holding frame 50 holds a lens group Lf1 formed of the doublet lenses CL1 to CL3 and the spacer rings SR1 and SR2, and a lens group Lf2 formed of the lenses L1 to L3 and the spacer rings SR3 and SR4. In this document, explanation will be given based on the assumption that the doublet lens is a single lens.

As illustrated in FIGS. 2 and 3, the lens holding frame 50 includes a first tube portion 51 formed in a tubular shape, and a second tube portion 52 with an outer diameter smaller than an outer diameter of the first tube portion 51. In addition, a through hole 53 with a wall surface formed in a stepped shape along a central axis direction is provided inside the lens holding frame 50. Specifically, the through hole 53 includes a first hole portion 53a extending from an end portion of the first tube portion 51 at a side different from a side connected to the second tube portion 52, a second hole portion 53b extending from the first hole portion 53a and having a diameter smaller than a diameter of the first hole portion 53a, a third hole portion 53c extending from an end portion of the second hole portion 53b at a side opposite to the first hole portion 53a and having a diameter smaller than the diameter of the second hole portion 53b, and a fourth hole portion 53d extending from an end portion of the third hole portion 53c at a side opposite to the second hole portion 53b and having a diameter larger than the diameter of the third hole portion 53c. The central axis described herein is a central axis of the through hole 53 and is an axis parallel to the optical axis C, for example.

Each of the lenses L1 to L3 is constituted by a single lens, and arranged at a predetermined position in the fourth hole portion 53d together with the spacer rings SR3 and SR4. The lenses L1 to L3 are arranged such that the lens L1, the spacer ring SR3, the lens L2, the spacer ring SR4, and the lens L3 are arranged in this order from the third hole portion 53c side, and a face of the lens L3 on the side opposite to the spacer ring SR4 is exposed to the outside from the lens holding frame 50. The lens L1 is a lens arranged on an end portion at a side different from an opening end side of the lens holding frame 50, and is in contact with a step portion formed by the third hole portion 53c and the fourth hole portion 53d.

Each of the doublet lenses CL1 to CL3 is constituted by a plurality of lenses attached to one another, and arranged at a predetermined position in the first hole portion 53a and the second hole portion 53b together with the spacer rings SR1 and SR2. The doublet lenses CL1 to CL3 are arranged such that the doublet lens CL1, the spacer ring SR1, the doublet lens CL2, the spacer ring SR2, and the doublet lens CL3 are arranged in this order from the third hole portion 53c side, and a face of the doublet lens CL3 on the side opposite to the spacer ring SR2 is exposed to the outside from the lens holding frame 50. The doublet lens CL1 is a lens arranged on an end portion at a side different from an opening end side of the lens holding frame 50, and is in contact with a step portion formed by the second hole portion 53b and the third hole portion 53c.

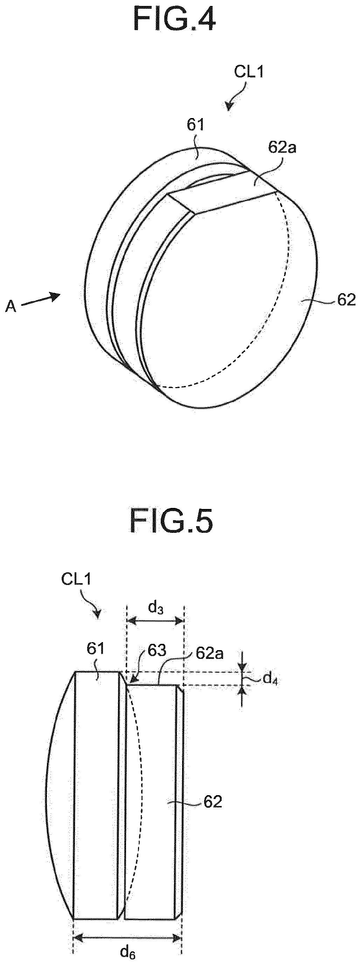

FIG. 4 is a perspective view schematically illustrating a configuration of a main part of the lens unit 5 of the endoscopic system 1 according to the embodiment of the present invention, and is a diagram for explaining a configuration of the doublet lens CL1. FIG. 5 is a diagram viewed from a direction of arrow A illustrated in FIG. 4. The doublet lens CL1 is constituted by two disc-shaped lenses attached to each other. Specifically, as illustrated in FIGS. 4 and 5, the doublet lens CL1 includes a first lens 61 and a second lens 62, which are attached to each other. A notch surface 62a is formed on the second lens 62 by cutting a part of an outside edge in a direction perpendicular to a radial direction. In the doublet lens CL1, the first lens 61 and the second lens 62 are attached to each other such that circumferential surfaces are aligned, and a step portion 63 having a stepped shape along the direction of the optical axis C is formed by a part of the bonding surface of the first lens 61 and the notch surface 62a of the second lens 62. In the embodiment, the pin 54 and the step portion 63 serve as an assembly error prevention unit.

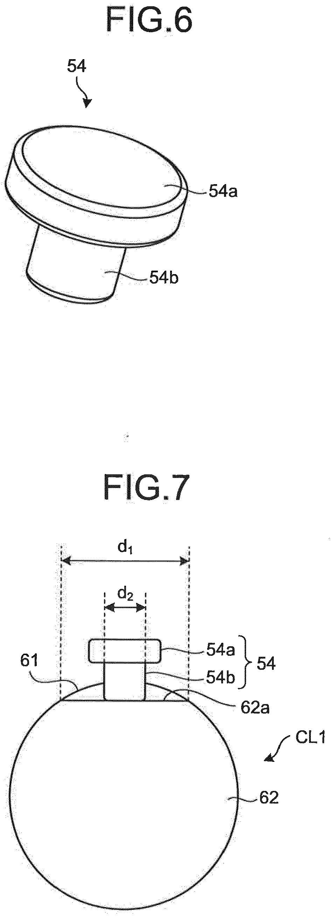

FIG. 6 is a perspective view schematically illustrating a main part of the lens unit 5 of the endoscopic system 1 according to the embodiment of the present invention, and is a diagram for explaining a configuration of the pin 54. The pin 54 includes a disc-shaped head portion 54a, and a cylindrical-shaped extending portion 54b extending from one surface of the head portion 54a and having a diameter smaller than a diameter of the head portion 54a. The pin 54 is provided so as to be freely inserted in and removed from a hole portion 52a formed on the second tube portion 52, and a distal end portion of the extending portion 54b protrudes from a wall surface of the second hole portion 53b. In the embodiment, a protruding portion of the pin 54 (the extending portion 54b) from the wall surface of the second hole portion 53b serves as a convex portion.

FIG. 7 is a schematic diagram for explaining a configuration of a main part of the lens unit 5 of the endoscopic system 1 according to the embodiment of the present invention, and is a schematic diagram illustrating the doublet lens CL1 and the pin 54 viewed from the direction of the optical axis C in the lens unit 5 illustrated in FIG. 3. Assuming that a length of the notch surface 62a of the second lens 62 in a direction perpendicular to the thickness direction and the radial direction is denoted by d₁, and a diameter of the extending portion 54b of the pin 54 (a length corresponding to the optical axis direction of the doublet lens CL1) is denoted by d₂, the length d₁ and the diameter d₂ have a relation of d₂ < d₁. In addition, assuming that a thickness of the second lens 62 is denoted by d₃ (see FIG. 5), the diameter d₂ and the thickness d₃ have a relation of d₂ < d₃. Therefore, the protruding portion (convex portion) of the extending portion 54b from the wall surface of the second hole portion 53b protrudes into a space formed by an inner circumferential surface of the lens holding frame 50 (the second hole portion 53b) and the step portion 63 and is housed in the space when properly assembled. The thickness d₃ corresponds to a length of a step of the step portion 63 in the direction of the optical axis C.

Furthermore, assuming that a maximum length of the step portion 63 (which is a height of the step in a direction perpendicular to the direction of the optical axis C of the doublet lens CL1, i.e., a maximum difference between the lengths of the first lens 61 and the second lens 62 in the radial direction) is denoted by d₄, and a protruding length of the pin 54 protruding from the wall surface of the second hole portion 53b is denoted by d₅, the maximum height d₄ and the protruding length d₅ have a relation of d₅ ≤ d₄.

Referring back to FIG. 3, in the properly assembled lens unit 5, the pin 54 is housed in a space formed by the step portion 63 of the doublet lens CL1 and a step portion formed by the second hole portion 53b and the third hole portion 53c. In this case, the notch surface 62a and a distal end of the extending portion 54b may be in contact with each other or may be located close to each other without contact.

When the lenses L1 to L3, the doublet lenses CL1 to CL3, and the spacer rings SR1 to SR4 are to be arranged in the lens holding frame 50, the lenses L1 to L3, the doublet lenses CL1 to CL3, and the spacer rings SR1 to SR4 are dropped into the through hole 53 in sequence. In this case, the doublet lenses CL1 to CL3 and the spacer rings SR1 and SR2 are dropped such that the doublet lens CL1, the spacer ring SR1, the doublet lens CL2, the spacer ring SR2, and the doublet lens CL3 are dropped in this order from the third hole portion 53c side to the first hole portion 53a and/or the second hole portion 53b.

When properly assembled, the doublet lens CL1 is positioned by contact with the step portion formed by the second hole portion 53b and the third hole portion 53c, without interfering with the pin 54 with the aid of the step portion 63.

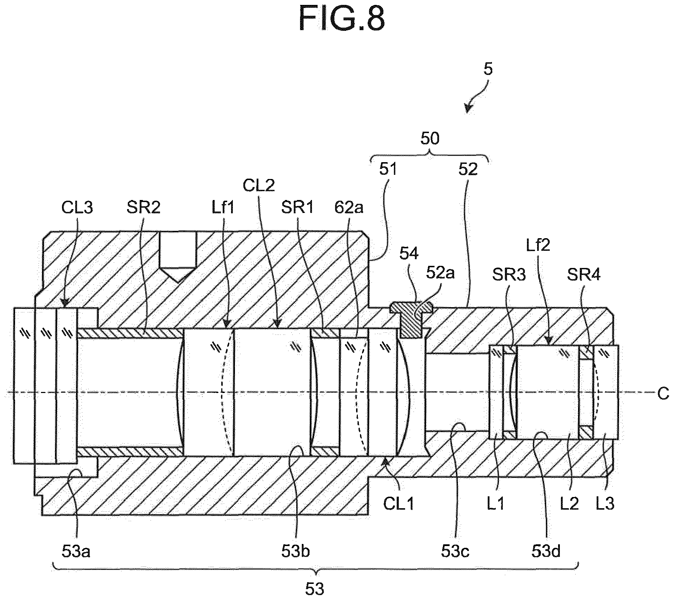

In contrast, when erroneously assembled, in particular, when the doublet lens CL1 is dropped with the front and back sides (one side and the other side in the optical axis direction) upside down, the front side of the first lens 61 comes in contact with the pin 54. FIG. 8 is a partial cross sectional view schematically illustrating the configuration of the lens unit 5 of the endoscopic system 1 according to the embodiment of the present invention, and is a diagram illustrating a case of an assembly error.

When the doublet lens CL is dropped into the through hole 53 while the first lens 61 is positioned at the side of the third hole portion 53c in the doublet lens CL1, the first lens 61 is positioned by contact with the pin 54. In this case, the first lens 61 is caught at the pin 54 and prevented from coming in contact with the step portion formed by the second hole portion 53b and the third hole portion 53c, so that the first lens 61 is not arranged in the position illustrated in FIG. 3. If the doublet lenses CL2 and CL3 and the spacer rings SR1 and SR2 are dropped while the doublet lens CL1 is erroneously arranged, the doublet lenses and the spacer rings are arranged at positions shifted by the deviation amount of the doublet lens CL1. For example, when it is designed such that the end face of the doublet lens CL3 is aligned with the end face of the lens holding frame 50 after the doublet lenses CL1 to CL3 and the spacer rings SR1 and SR2 are arranged through correct assembly as illustrated in FIG. 3, and if the components are erroneously assembled, a part of the doublet lens CL3 protrudes from the end face of the lens holding frame 50 (see FIG. 8). A user can determine whether the doublet lens CL1 is erroneously assembled by checking the position of the doublet lens CL3 at the time of assembly. In addition, it is possible to determine whether the doublet lens CL1 is erroneously assembled by checking an amount of protrusion of the spacer ring SR2 from the second hole portion 53b or by checking reflection of light from the doublet lens CL1 by pulling out the pin 54 from the hole portion 52a before dropping the doublet lens CL3.

According to the embodiment of the present invention as described above, the step portion 63 is provided by cutting a part of the doublet lens CL1, and the pin 54 is provided so as to protrude inside the second hole portion 53b in the lens unit 5 such that the doublet lens CL1 and the pin 54 interfere with or do not interfere with each other depending on the dropping direction of the doublet lens CL1. Therefore, the position of the doublet lens CL1 changes depending on the dropping direction, and the positions of the doublet lenses CL2 and CL3 and the spacer rings SR1 and SR2 to be subsequently dropped also change, so that it is possible to easily determine an assembly error of the lens when the lens unit 5 is assembled. Consequently, it is possible to determine an assembly error of the lens before the lens unit 5 is assembled inside the operating unit 22 before completion of the endoscope, and it is possible to prevent a conventionally performed operation of decomposing the endoscope and replacing the lens because of detection of an assembly error of the lens after completion of the endoscope.

### (Modification of Embodiment)

While a case has been explained based on the assumption that the single step portion 63 with the notch surface 62a is provided in the embodiment described above, the present invention is not limited to this example. For example, it may be possible to provide two or more step portions to increase the flexibility of assembly in a case where the doublet lens rotates around the optical axis.

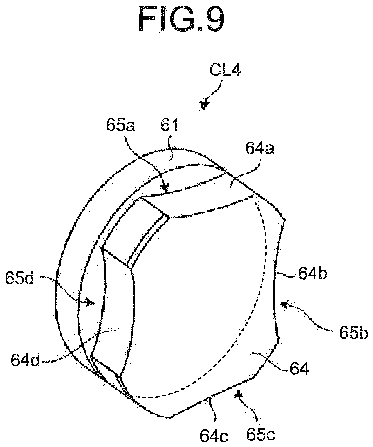

FIG. 9 is a perspective view schematically illustrating a configuration of a main part of a lens unit of an endoscopic system according to a modification of the embodiment of the present invention. A doublet lens CL4 illustrated in FIG. 9 includes a second lens 64 having four notch surfaces (notch surfaces 64a to 64d) and step portions 65a to 65d corresponding to the respective notch surfaces 64a to 64d, instead of the second lens 62 of the doublet lens CL1. The doublet lens CL4 can be properly assembled even when the doublet lens CL4 rotates by 90 degrees around the central axis (the optical axis C).

Furthermore, while the doublet lens CL1, which is a lens arranged on an end portion at a side different from the opening end side of the lens holding frame 50, has been described as an example in the embodiment and the modification described above, the disclosed technology may be applied to a lens arranged at a different position. In this case, a slit that does not interfere with the pin 54 is formed in a lens or a spacer ring to be located on a deeper side in the dropping direction than a target lens for which an assembly error is to be determined. To determine the assembly error from a firstly dropped lens or spacer ring, it is preferable to provide an assembly error prevention unit (the pin 54 and the step portion 63) on the lens or the spacer ring on the deepest side in the dropping direction.

Moreover, while a case has been explained in which the lens holding frame 50 holds a lens group including a plurality of lenses and spacer rings in the embodiment and the modification described above, it may be possible not to include a spacer ring or to hold a single lens.

Furthermore, while a case has been explained in which each of the doublet lenses CL1 and CL4 is formed of two lenses attached to each other in the embodiment and the modification described above, the disclosed technology may be applied to a single lens or a doublet lens formed of three or more lenses attached to one another. In this case, particularly in a case of a single lens, a step portion having a notch surface is provided such that the length of the step in the optical axis direction becomes shorter than the thickness of the lens. For example, in the doublet lens CL1 illustrated in FIG. 5, assuming that a maximum thickness at the outside edges of the first lens 61 and the second lens 62 in the direction of the optical axis C is denoted by d₆, the length d₃ of the step in the direction of the optical axis C (corresponding to the thickness of the second lens 62) and the maximum thickness d₆ have a relation of d₃ < d₆.

Moreover, while a case has been explained in which the pin 54 is inserted in the second tube portion 52 in advance in the embodiment and the modification described above, it may be possible to remove the pin 54 from the hole portion 52a before the doublet lens CL1 is dropped, and insert the pin 54 after the doublet lens CL1 is dropped. In this case, even when the notch surface 62a of the doublet lens CL1 is slightly inclined in the insertion direction of the pin 54, the pin 54 comes in contact with the notch surface 62a and causes the doublet lens CL1 to rotate. Therefore, even when the doublet lens CL1 is dropped while slightly rotating about the central axis, it is possible to perform adjustment to obtain a properly assembled state without interference between the pin 54 and the doublet lens CL1 in the direction of the optical axis C, by inserting the pin 54.

Furthermore, while a case has been described in which the pin 54 is provided separately from the second tube portion 52 and a part of the pin 54 (the distal end portion of the extending portion 54b) protrudes from the wall surface of the second hole portion 53b in the embodiment and the modification described above, it may be possible to integrate the second tube portion 52 and the pin 54, and provide a convex portion that protrudes inwardly from the second hole portion 53b.

Moreover, while a case has been described in which the endoscope 2 is an ultrasound endoscope, a part of which is insertable into a subject and which has a function to transmit an ultrasound pulse toward a body wall inside the subject, to receive ultrasound echoes, and to output an echo signal, and a function to capture an image of the inside of the subject and to output an image signal in the embodiment and the modification described above, it may be possible to employ an endoscope having only the function to capture an image of the inside of the subject and output an image signal, or an endoscope having only the function to transmit an ultrasound pulse toward a body wall inside the subject, to receive ultrasound echoes reflected from the subject, and to output an echo signal, for example.

In this manner, the present invention may include various forms of embodiments without departing from the technical sprit and scope of the general invention concept as defined in the appended claims of this invention.

### Industrial Applicability

As described above, the lens unit and the endoscope according to the present invention are useful for easily determining an assembly error of the lens during assembly.

### Reference Signs List

1 ENDOSCOPIC SYSTEM
2 ENDOSCOPE
3 IMAGE PROCESSING APPARATUS
4 LIGHT SOURCE DEVICE
5 LENS UNIT
21 INSERTION PORTION
22 OPERATING UNIT
23 UNIVERSAL CODE
24 CONNECTOR
50 LENS HOLDING FRAME
51 FIRST TUBE PORTION
52 SECOND TUBE PORTION
53 THROUGH HOLE
54 PIN
61 FIRST LENS
62 SECOND LENS
62a, 64a to 64d NOTCH SURFACE
63, 65a to 65d STEP PORTION
CL1 to CL4 DOUBLET LENS
L1 to L3 LENS
Lf1, Lf2 LENS GROUP
SR1 to SR4 SPACER RING

## Claims

1. A lens unit comprising:
a plurality of lenses;
a lens holding frame having a tubular shape and configured to hold the plurality of lenses inside thereof;
a step portion provided in any of the plurality of lenses and having a stepped shape along a direction of an optical axis of the lens; and
a convex portion protruding from an inner circumferential surface of the lens holding frame into a space formed by the inner circumferential surface of the lens holding frame and the step portion, in accordance with a position of the lens in which the step portion is formed.

2. The lens unit according to claim 1, wherein a length of the convex portion corresponding to the direction of the optical axis is shorter than a length of a step of the step portion in the direction of the optical axis.

3. The lens unit according to claim 1, wherein a protruding length of the convex portion from the inner circumferential surface of the lens holding frame is equal to or shorter than a height of a step of the step portion in a direction perpendicular to the optical axis.

4. The lens unit according to claim 1, wherein the convex portion is provided so as to be freely inserted in and removed from an opening portion formed on the lens holding frame.

5. The lens unit according to claim 1, wherein the lens includes a plurality of the step portions.

6. The lens unit according to claim 1, wherein the convex portion is formed in a certain position in accordance with a lens that is arranged on an end portion at a side different from an opening end side of the lens holding frame in a lens group formed of the plurality of lenses and spacer rings provided between adjacent ones of the lenses.

7. An endoscope having an insertion portion inserted into a subject, the endoscope comprising:
the lens unit according to claim 1; and
an image sensor configured to receive light that has passed through the lens unit and convert the light into an electrical signal.
